# EUROPEAN PATENT APPLICATION

(11) **EP 2 484 750 A1**
(43) Date of publication of application: **08.08.2012**
(21) Application number: 11153590.2
(22) Date of filing: 07.02.2011
(51) Int. Cl.: C12M 1/34

(54) **Monitoring system for cell culture**

(71) Applicant: Ecole Polytechnique Fédérale de Lausanne (EPFL), 1015 Lausanne (CH)
(72) Inventor: Meurville, Eric, FR-25300, CHAFFOIS (FR); Barrandon, Yann, CH-1005, LAUSANNE (CH); Abou-Jaoude, Georges, CH-1806, SAINT-LEGIER (CH)
(74) Representative: reuteler & cie SA

(57) **Abstract**

Cell culture environment monitoring system (6) for monitoring parameters relevant to cell growth in at least one culture dish (4) containing a cell growth medium (14), including at least one sensing device (22, 22') configured to measure environmental parameters relevant to cell growth, and a tray (24) supporting said at least one culture dish. The sensing device is configured for mounting inside said culture dish at least partially within said cell growth medium, and comprises an RFID transponder (34). The tray (24) comprises an RFID base station (44) configured to interrogate the RFID transponder to obtain measurements of said parameters relevant to cell growth.

## Description

### Field of the Invention

The invention relates to a system for monitoring a culture environment for the growth of mammalian and non-mammalian cells.

### Background of the Invention

There is a need for academic researchers, applied clinicians or industrial scientists to have an incubating system that produces high yields while decreasing labour demands over traditional cell culture devices and without impact on culture process protocols.

For instance, stem cells generate great hopes for disease modelling, drug discovery or as a therapeutic tool for regenerative medicine. Stem cells can be obtained from different sources during the life of an individual (embryonic, cord blood, various adult tissues), and each type of stem cells has advantages and limits. Major advances in stem cell cultivation has provided the capability to robustly manipulate stem cell fate *ex vivo,* as demonstrated by the genetic reprogramming of adult stem cell to ground pluripotency (induced pluripotent stem cells - iPS) or by the reprogramming of thymic epithelial cells to multipotent stem cells of the hair follicle in response to an inductive skin microenvironment. However, manipulation of stem cell fate necessitates a strict control of culture conditions that can affect stem cell behavior. Current technology only provides approximate read-out and not in a real-time fashion.

Controlling the culture conditions of embryos is also a key to the success of assisted reproduction procedures aiming to single blastocyst implantation, in particular to decrease the probability for multiple pregnancies.

In contrast to conventional two-dimensional culture vessels, there is an advantage for certain applications in growing cells in suspension or adherent cultures, including organotypic culture, which involves growing cells in a three-dimensional environment that is biochemically and physiologically more similar to *in vivo* tissue.

Culture dishes are typically made of a transparent plastic, sometimes glass, and come in standard sizes. There would be an advantage in providing a culture system that is compatible or may be used with commercial off-the-shelf culture dishes.

In WO1998020108A1 an apparatus for holding cells comprises a mechanism for incubating cells, having a dynamically controlled environment in which the cells are grown, which are maintained in a desired condition and in which cells can be examined while the environment is dynamically controlled and maintained in the desired condition. The system is dedicated to individual cell analysis in a dynamically controlled environment but not to cell growth medium monitoring even if this functionality is mentioned (dynamically controlled environment).

In WO2007120619A2 an incubation condition monitoring device has a reader unit to measure selected characteristics within an incubator. The reader unit transmits the information to a receiver/transmitter within the incubator to receive the measurements and to transmit the measurements of the selected characteristics to a data logger outside the incubator. A monitor and display system monitors and display the measurements of the selected characteristics. The selected characteristics within the incubator can be temperature and pH. A cuvette contains a sample of the fluid which is the same as that in the culture dish. The measurement is thus indirect and may not correspond to the actual cell culture environment.

In US2006003441A1 a cell culture system includes a monitoring system with predefined sensor modules. By means of this monitoring system, parameters in the relevant cell culture chamber can be measured using accordingly assigned sensors for the duration of a test. For this purpose, the monitoring system is connected to the individual cell culture chambers. The parameters measured by the sensors are transmitted by the monitoring system via a line to the computer-controlled monitoring and control system for further processing. The system however uses dedicated dishes, not commercially available Petri dishes.

### Summary of the Invention

An object of this invention is to provide a system for monitoring a culture environment for the growth of mammalian and non-mammalian cells that enables economical yet high yield growth of cells and that has no or negligible impact on culture process protocols.

It is an advantage to provide a system for monitoring a culture environment for the growth of cells that requires minimal handling by personnel.

It is an advantage to provide a system for monitoring a culture environment for the growth of cells that is reliable and safe.

It is an advantage to provide a system for monitoring a culture environment for the growth of cells that reduces processing of culture growth parameters and data.

It is an advantage to provide a system for monitoring a culture environment for the growth of cells that allows easy adjustment of culture growth parameters to increase yield.

Objects of this invention have been achieved by providing a cell culture environment monitoring system according to claim 1.

Objects of this invention have also been achieved by providing a cell culture growth system according to claim 14.

Disclosed herein is a cell culture growth system and a culture environment monitoring system for monitoring parameters relevant to cell growth in at least one culture dish containing a cell growth medium, including at least one sensing device configured to measure environmental parameters relevant to cell growth comprising an RFID transponder, and a tray supporting said at least one culture dish comprising an RFID base station configured to interrogate the RFID transponder of the sensing device.

The sensing device is configured for mounting inside the culture dish and may be immersed partially or totally within said cell growth medium for measuring at least one parameter within the cell growth medium. The sensing device, or an additional sensing device may also be positioned outside or partially outside the cell growth medium to measure parameters in the gaseous environment in the immediate vicinity of the culture medium within the culture dish.

The tray may advantageously comprise a support base configured to support and position thereon a plurality of culture dishes. More than one, or all of the culture dishes may have one or two sensing devices mounted therein.

The RFID base station includes an antenna that may advantageously be mounted in or on the support base configured to enable communication between the RFID base station and the plurality of sensing devices positioned in the plurality of culture dishes. The antenna may comprise a conductor loop surrounding the plurality of culture dishes configured for near-field communication with the sensing devices, the antenna embedded in or mounted on the support base.

The support base may advantageously comprise optical inspection ports positioned below the culture dishes to allow passage of light through the culture dish and growth medium for optical inspection or testing without having to remove the culture dish from the tray.

The RFID base station may include a signal processing circuit that includes an RFID interrogator, a microprocessor, a portable power source such as a battery, a communications interface configured for wireless and/or hardwire link to an external computing system and optionally a memory for storing or logging data received from the sensing devices.

The sensing device advantageously comprises a plurality of sensors responsive to different parameters relevant to cell growth, which may include temperature, pH, Ca⁺⁺, CO₂ glucose and other cell nutrients, O₂ and light.

The sensing device may further include a microprocessor and energy collecting and/or storage means for short term power supply of the RFID transponder, microprocessor and sensors.

The sensing device comprises a support or base on which, or within which, the sensors and signal processing circuitry are mounted. In an advantageous embodiment, the support comprises an adhesive base configured to allow the sensing device to be stuck on an inside surface of the culture dish and immersed partially or totally in the cell culture growth medium. The sensing device may be generally in the form of a thin sticker or label having a height less than 5mm, possibly less than 3mm, that allows it to be stuck on the bottom wall of a conventional Petri dish and completely covered by the growth medium contained in the Petri dish, or stuck on the underside of the cover of the Petri dish without contacting the cell growth medium when the cover is positioned on the container part of the Petri dish.

In a variant, the sensing device may comprises sensor probes that extend at different lengths, certain said sensor probes configured for insertion in the cell growth medium and other said sensor probes configured to remain outside of the growth medium such that parameters within the growth medium and the gaseous environment surrounding the growth medium can be measured by the same sensing device. The sensing device may have mechanical fixing means such as a clip or hook or clasp for fixing to the culture dish side wall.

Advantageously, the system according to the invention enables non invasive continuous monitoring of multiple parameters relevant to cell growth (e.g. temperature, light, CO₂, O₂, pH, glucose concentration and other nutrients), to achieve high cell growth rates and yields in a safe, reliable and economical manner. In addition, the cells can further be examined by standard techniques, e.g. by standard imaging techniques, in this wirelessly monitored growth medium.

Further objects and advantageous aspects of the invention will be apparent from the claims, following detailed description and accompanying figures.

### Brief Description of the drawings

Figure 1 is a schematic diagram illustrating components of a system for monitoring a culture environment for the growth of cells according to an embodiment of the invention;
Figure 2 is an illustration of a cell culture growth system including a cell culture environment monitoring system according to an embodiment of the invention;
Figures 3a to 3c are respectively perspective, top and side views of a cell culture environment monitoring system according to an embodiment of the invention;
Figures 4a and 4b are respectively perspective and side views of a cell culture dish and sensing devices according to an embodiment of the invention;
Figures 5a to 5c are respectively perspective, top and side views of the sensing device of figures 4a, 4b;
Figures 6a, 6b are similar to figures 4a, 4b except that a sensing device according to another embodiment is illustrated;
Figure 7 is a variant of the embodiment of figure 6a, 6b where the sensing device is mounted on a cover of the culture dish rather than the base of the culture dish;
Figure 8 is a simplified circuit diagram of an embodiment of a sensing device according to the invention;
Figure 9 is a simplified diagram of an embodiment of a biosensor of a sensing device according to an embodiment the invention;
Figure 10 is a simplified circuit diagram of a sensing device including a control circuit of the biosensor of figure 9 according to an embodiment of the invention;
Figure 11 is a simplified circuit diagram of a sensing device including a control circuit of a biosensor according to another embodiment of the invention.

### Detailed Description of embodiments of the invention

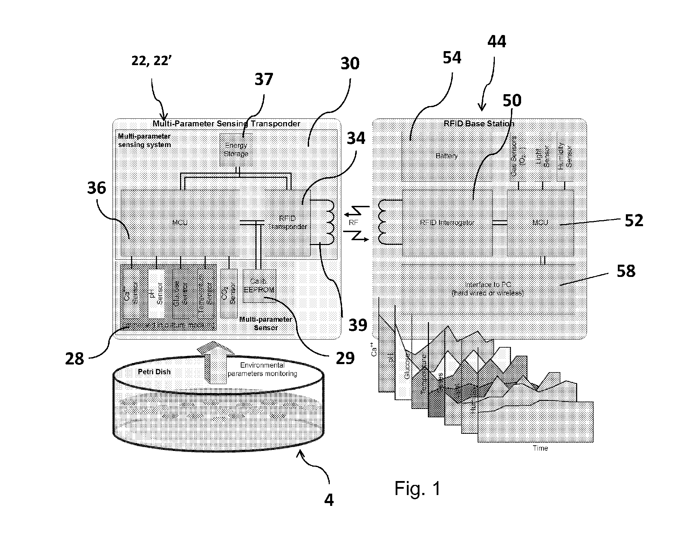

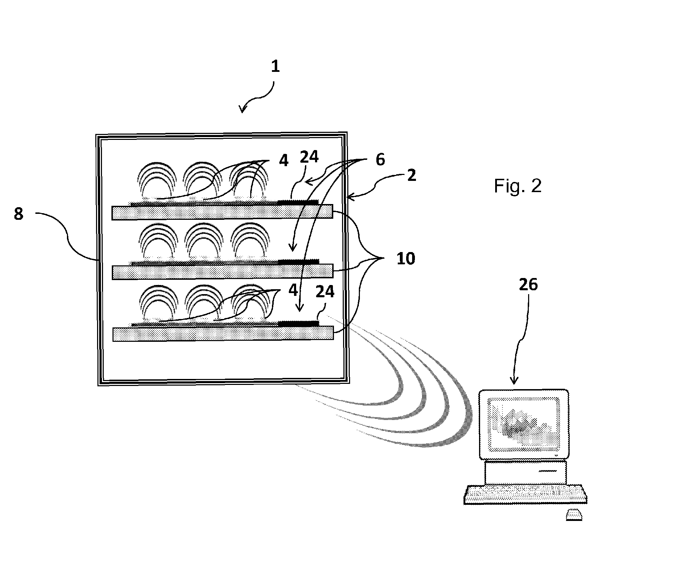

Referring to the figures, in particular first to figures 1 and 2, a cell culture growth system 1 comprises an incubator 2 in which one or more culture dishes 4 are received, and a cell culture environment monitoring system 6 optionally connected to a computing and user interface system 26. The incubator 2 is *per* se well-known and comprises an enclosure 8 in which there may be one or more shelves 10 for placing culture dishes, the environment inside the incubator being controlled by a control system that may typically control the temperature, humidity, carbon dioxide, oxygen and other gaseous components inside the incubator.

Culture dishes 4, which include well-known so-called "Petri dishes", are widely commercially available in various standard sizes, often made of a transparent plastic, sometimes of glass, comprising a recipient or container part 18 and a cover part 20 to cover the open end of the container part 18. The container part 18 is partially filled with a cell culture growth medium 14, for example in the form of a gel containing various nutrients, the quantity and composition thereof being adapted to the specific type of cells to be grown in the culture dish. The culture dishes and growth mediums are *per* se well-known in the art.

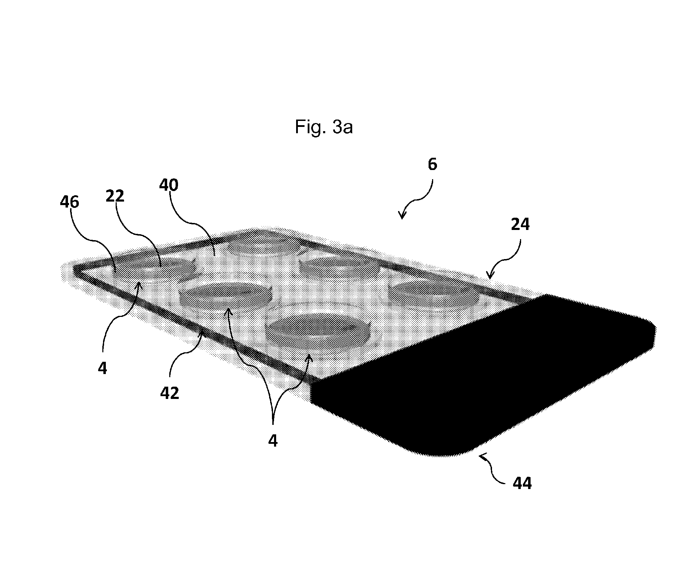

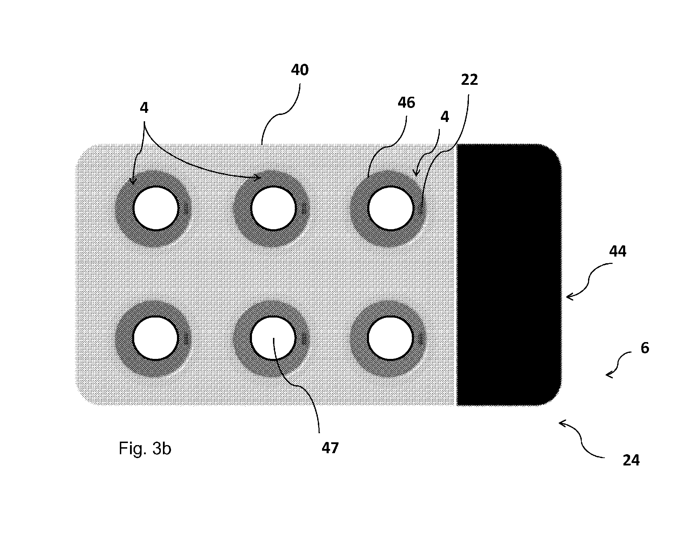

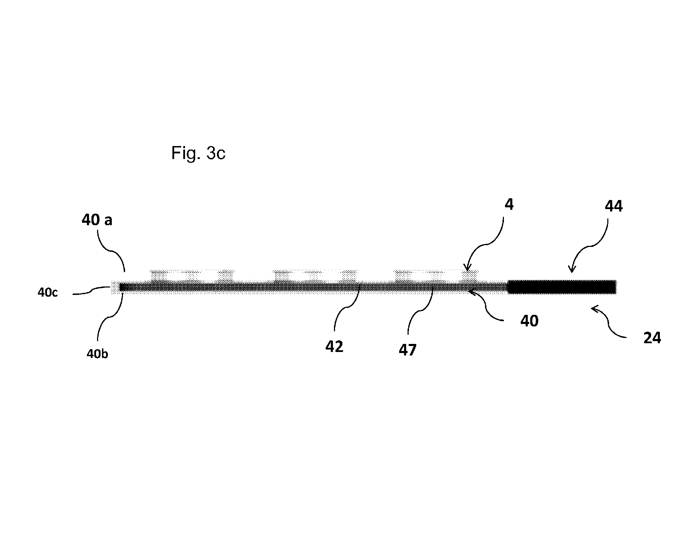

A cell culture environment monitoring system 6 according to an embodiment of this invention includes one or more sensing devices 22 mounted in each culture dish and a communications and support tray 24 that communicates with one or more sensing devices 22. Referring to figures 3a to 3c, the communications and support tray comprises a support base 40 with culture dish positioning means 46 for positioning one or a plurality of culture dishes 4 thereon, a radio frequency identification (RFID) base station 44, and an antenna 42 that allows communication between the RFID base station 44 and the sensing devices 22 positioned in each of the culture dishes 4.

The dish positioning means 46 may simply be in the form of recesses, for example circular recesses within the support base 40 each configured to snugly receive the periphery of the base of a container part 18. Instead of recesses, other positioning means such as protuberances projecting from the support base for positioning the culture dish container part thereon may be provided. The support base 40 may comprise a plurality of positioning means for positioning culture dishes of different sizes, for example by providing a large recess and co-axially therein a further smaller recess (not shown) within the base of the large recess for a smaller container. This enables the communications and support tray 24 to be used with culture dishes of different sizes. The communications and support tray advantageously comprise a plurality of culture dish positioning elements, for example 4, 6, 8 or more and 2, 3, 4 or more rows and/or columns of a size suitable for positioning on a shelf inside a conventional incubator.

The antenna 42 may be in the form of a conductor loop embedded in the support base 40 surrounding culture dishes 4, configured to allow near field RFID communication with the sensing devices 22 positioned in the culture dishes 4. The antenna conductor 42 may thus form a loop close to, or on the outer contour of the support base 4 surrounding the culture dishes. The antenna 42 may be configured as a single loop or a plurality of windings and may be formed as a coil embedded within the support base 40 or formed on a surface of the support base, either a top or bottom surface 40a, 40b or a lateral surface 40c. The antenna may also be in the form of a separate component that is mounted on the support base 40 by various known fixing means such as bonding with an adhesive, mechanical clasp or clipping means, or welded for example by ultrasonic welding.

The support base 40 may advantageously comprise optical inspection ports 47 positioned within the recess or positioning means of the culture dishes below the container part 18 base wall to allow passage of light through the culture dish and growth medium for optical inspection or testing, for example by means of microscope, spectrometer or other optical testing systems, either automatically or by manual manipulation, without removing the culture dishes from the communications and support tray. The optical inspection port may be formed by a passage or hole through the support base, corresponding to only a small portion of the surface area below the culture dish, or covering almost all the surface of the culture dish which is then supported on the base essentially only at the periphery for example. Alternatively, instead of a hole through the support base, the support base may comprise an optically transparent material, such as a transparent plastic material, or have a transparent portion for the inspection port underneath the culture dish.

Referring to figures 1, 2 and 3a to 3c, the RFID base station comprises a signal processing circuit that includes an RFID interrogator 50, a microprocessor 52, a power source 54, such as a battery that is rechargeable and/or replaceable, a communications interface 58 in the form for instance of a data transceiver or communication port for wireless and/or hardwire link to an external computing and user interface system 26, and optionally a memory for storing or logging data received from the sensing devices. The RFID interrogator communicates via the antenna 42 with the RFID transponder circuits 34 of the sensing devices 22. RFID communication techniques are *per* se well-know and need not be described in detail herein.

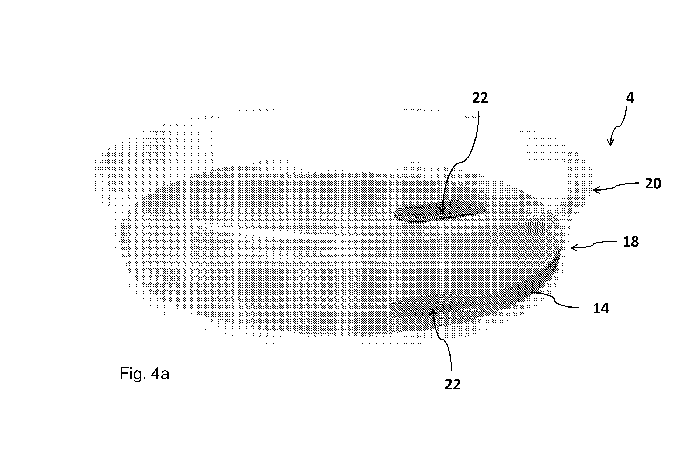

Referring to figures 4a, 4b, figures 5a to 5c, and figure 8, a sensing device 22 according to embodiment of this invention comprises one or more single parameter sensors or one or more multi parameter sensors 28, signal processing circuitry 30 comprising an RFID transponder 34 with a coil 39, a microprocessor 36, and energy collecting and/or storage means 37 for short term power supply of the RFID transponder, microprocessor and sensors. The sensing device 22 further comprises a support or base 32 on which, or within which, the sensors and signal processing circuitry and microprocessors are mounted. The support may comprise a plastic film, for instance of PVC or PET, or base made of another material that has fixing means for fixing the sensor devices to a surface or wall of the culture dish such that the sensors 28 are positioned within the culture dish.

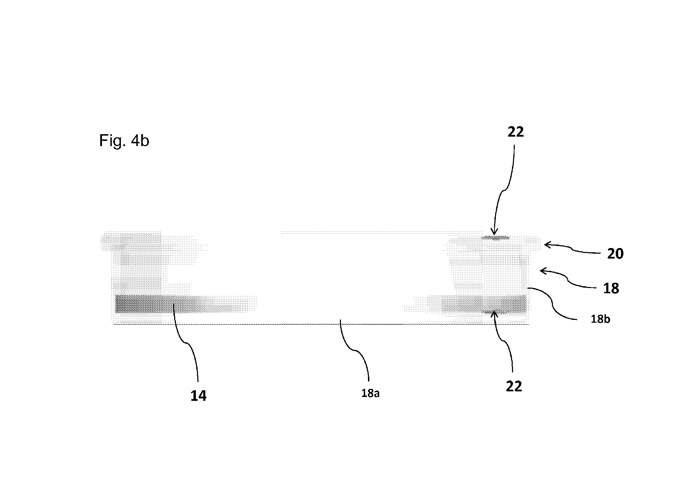

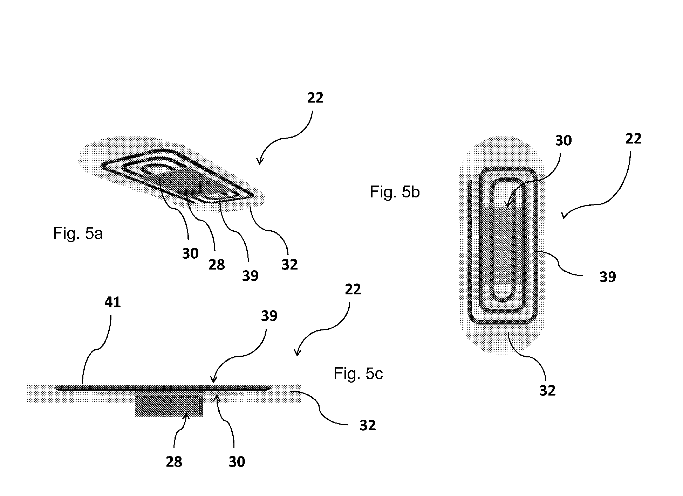

In a first embodiment illustrated in figures 5a to 5c, the fixing means comprise an adhesive base 32 that allows the sensing device 22 to be stuck on an inside surface of a bottom wall 18a or side wall 18b of the container part 18 of the culture dish, immersed in the cell culture growth medium 14 to measure parameters within the cell culture growth medium. Another sensing device may be mounted on a portion of surface within the culture dish outside of the culture medium, for example against an upper inner side wall of the container part or against an inner side of the top wall of the cover part 20 in order to measure parameters of the gaseous environment outside but surrounding the cell culture growth medium. A pair of sensing devices may for example be mounted in a culture dish, one on the container bottom wall 18a and the other on the cover part 20 as illustrated in figures 4a and 4b, allowing to measure a temperature gradient between the bottom wall and the cover for instance.

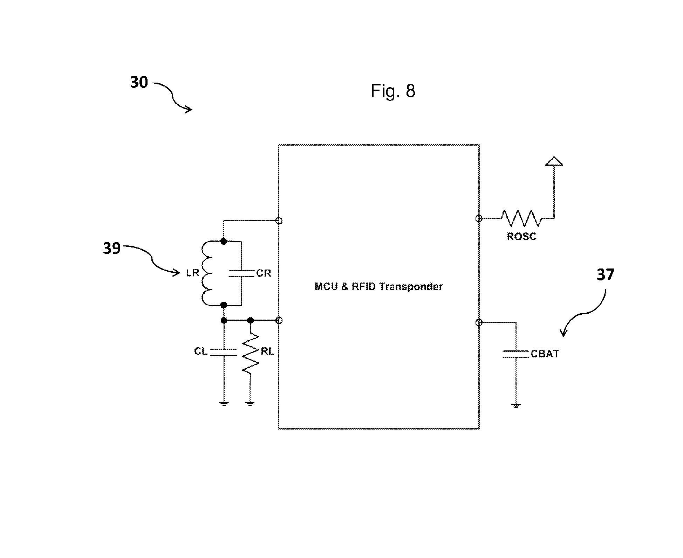

The RFID transponder 34 may, as is *per* se known in RFID transponders, comprise a conductive coil 39 that acts as a resonance coil L_{R} of the RFID transponder to capture and transmit wireless signals to the RFID base station 44 via the antenna 42. Referring to figure 8, the components of an embodiment of the RFID transponder circuit include as illustrated:
- L_{R}: resonance coil
- C_{R}: resonance capacitor
- C_{L}: charge storage capacitor
- R_{L}: termination resistor
- C_{BAT}: block capacitor for supply voltage
- R_{OSC}: current source for the internal oscillator

The RFID transponder may comprise:
One culture dish may be monitored by one or more multi-parameter transponders, each being identified by a unique serial number.

It is also possible, in the case of multi-dish configuration per tray (as illustrated in figs 3a-3c) to monitor only a single dish, or a plurality but not all on the tray. A multi-parameter sensor may measure various parameters such as Ca⁺⁺, pH, glucose and temperature inside the culture medium and parameters outside but in the close neighborhood of the culture medium such as CO₂ and temperature.

The sensing devices may for instance comprise a near field RFID transponder operating at a frequency configured to avoid any interference with cell metabolism, preferably less that 30MHz, for instance 125KHz, 134.2KHz, 13.56MHz or 27MHz, more preferably 125KHz or 134.2KHz, with an RFID antenna average diameter or width in the range of 5 to 20mm, for instance approximately 10mm.

The RFID base station interrogator 50 may comprise a selective address mechanism, for instance one reader may address up to 255 transponders. The RFID base station 44 may further comprise a far field communication transceiver for example operating at a frequency such as 2.4GHz, for wireless communication with the computer system 26.

In a preferred embodiment, the sensing device 22, 22' comprises a plurality of sensors, including a temperature sensor, a pH sensor and analyte sensors which may include a glucose sensor and a calcium ion (Ca⁺⁺) sensor. Further sensors may be included that are relevant to the specific cells that are grown including for example sodium and/or potassium ion sensors. Further sensors may be included to measure light radiation in particular for measuring light energy and possibly also light spectrum that is applied on the cell culture either for promoting, reducing or stabilizing cell growth. There may also be sensors for measuring parameters of the gaseous environment that are relevant to cell growth or indicative of cell culture activity, in particular carbon dioxide and/or oxygen. Sensors may also be provided to measure humidity and other gaseous components in the culture dish close to the growth medium. The sensors may advantageously be configured to measure these parameters intermittently at regular or predetermined intervals, or on user initiated request, by corresponding interrogation by the RFID interrogator to form a quasi continuous or on demand multi parameter monitoring system. The RFID base station thus energizes the multi-parameter sensing devices and interrogates them periodically or at any predetermined or user activated time to upload measured environmental parameters.

Depending on the application, the data may be recorded in the signal processing circuit of the sensing device 22, 22', which may comprise a data logger function, in waiting for a further interrogation by the RFID base station. Otherwise, the data may be sent on the fly in real time towards the RFID base station, i.e. corresponding to an Interrogator function.

One RFID base station 44 may thus control several multi-parameter sensing devices, i.e. a single- as well as a multi-well configuration is possible. The RFID base station 44 may also comprise one or more sensing devices to measure overall environment parameters within the incubator or other environment surrounding the tray 24 and culture dishes 4, for instance to measure any one or more of the following parameters: temperature, O₂, CO₂, light and humidity.

The RFID base station may transmit by hard wiring (e.g. USB) or wirelessly (e.g. Bluetooth) collected data to a computing device 26 for display and processing purposes from the incubator where the culture batch is stored between manipulations. A logging of the selected parameters to provide an audit history throughout the cell culture cycle may be then enabled, and provide warnings if some of these parameters go outside of a specified range. The monitor and display system may include an alarm mechanism that provides an alarm (audible, visual, SMS or e-mail) when the measured characteristics are outside a selected range

The monitoring process may operate either when the culture batch is inside or outside the incubator, e.g. during the feeding process or the observation stage, when the dish is placed under an imaging system (e.g. microscope).

A normal RFID system is completely passive: the RFID base station sends a command to a RFID transponder, and the transponder answers, normally with its serial number. The sensing device transponder 22 is not connected to an internal power source: it is completely powered out of the RF field supplied by the RFID base station 44.

In addition to serial number, the RFID transponder 34 is able to control a multi-analyte sensing system: the transponder of the multi-parameter sensing device captures the radio-frequency power (125KHz, 134.2KHz, 13.56MHz or 27MHz) through the site antenna, energizes the sensor, performs the measurement, converts it to a digital value, and sends this information back to the RFID Base Station.

The on site microprocessor 36 of the sensing device 22, 22' may perform some compensation computations beforehand, stored in a calibration EEPROM 29 programmed in factory.

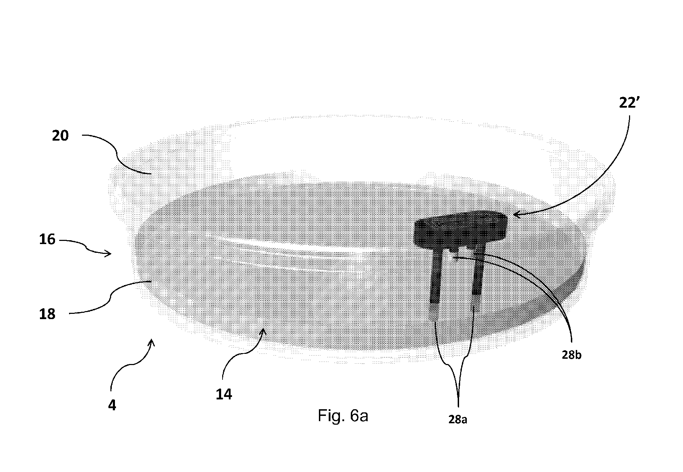

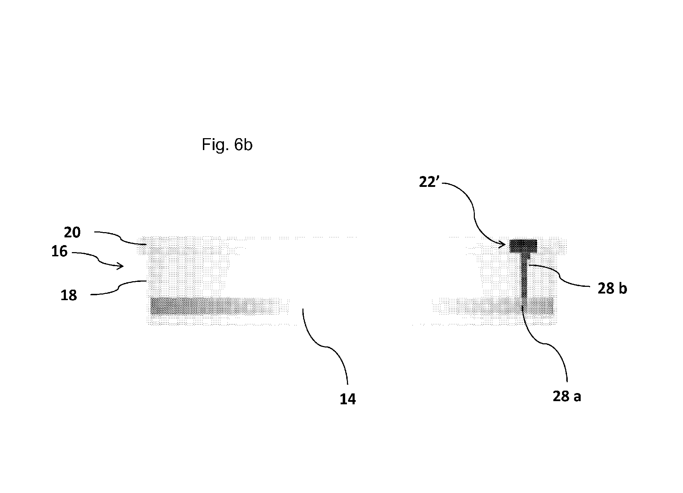

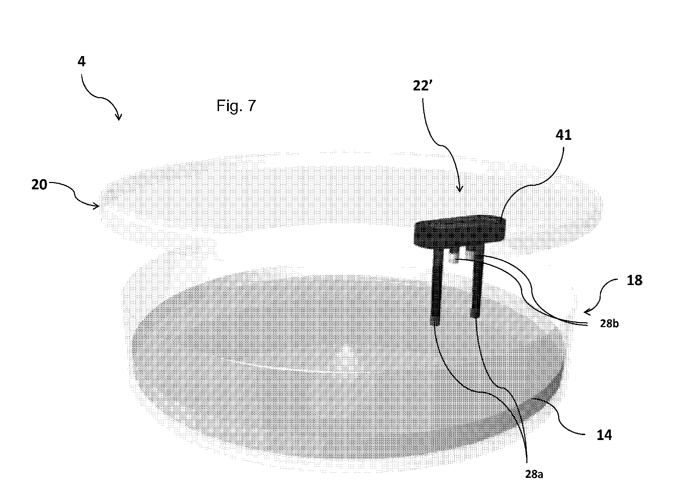

Referring to figures 6a, 6b and 7, another embodiment of a sensing device 22' is illustrated, the sensing device being configured to be fixed, for instance by means of a clip, to a side-wall of the container part 18 of the dish, or to the cover 20 by means of an adhesive (figure 7) outside of the culture medium. In this variant, the sensing device comprises sensor probes 28 that extend at different lengths, certain probes 28a inserted in the growth medium 14 and other sensor probes 28b outside of the growth medium such that parameters within the growth medium and the gaseous environment surrounding the growth medium can be measured with the sensor device 22'.

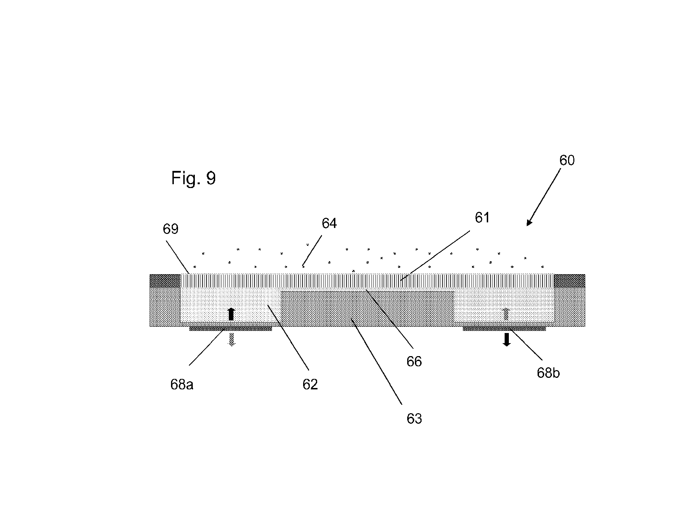

Examples of the functional and operational characteristics of the sensors comprised in the sensing devices 22, 22' are described below and in relation to figures 9 to 11.

Temperature may be measured by a temperature sensor generally internal to the microprocessor 36. For certain other parameters, two advantageous measurement methods allowing targeting minimal footprint and production costs compatible with disposability may be implemented as follows.

### Measurement based on viscosity change detection (e.g. glucose):

A chemico-mechanical method which aims at detecting viscosity changes of a solution with a selective affinity for the analyte of interest has been proposed in [Boss09] and [Boss11]. Referring to figure 9, an analyte sensor 60 that may be integrated in the sensing device 22, 22' may include a semi-permeable membrane 61 (for instance a free-standing AAO nanoporous membrance) that ensures that the analyte concentration in the sensitive solution 62 of the biosensor is similar to its concentration in the external solution 64 to analyze (in this case the culture cell growth medium 14 in the culture dish), and is comparable to the concentration observed in biological fluids. The determination of the viscosity of the sensitive solution is based on a micro-channel 66 which exhibits a resistance to the flow circulating through it. The sinusoidal actuation of an actuating diaphragm 68a (e.g. piezoelectric diaphragm) generates a flow through the micro-channel 66 which deflects a sensing diaphragm 68b (e.g. a piezoelectric diaphragm), inducing a voltage which can be recorded. The phase shift between the applied voltage and the sensing piezoelectric diaphragm deflection is a measurement of the viscosity of the sensing fluid. For instance, the sensitive solution encapsulated into the sensor may exhibit a selective affinity to glucose. In addition, in view of cost reduction and large scale production, such a sensor may be achieved by MEMS fabrication techniques in a semiconductor substrate 63. An anti-biofouling coating 69 may be deposited on the semi-permeable membrane 61 to prevent tissue growth on the semi-permeable membrane.

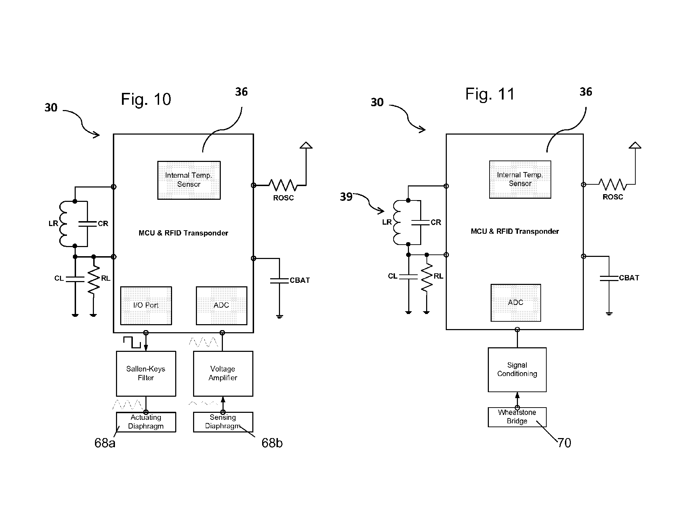

Referring to figure 10, to control the actuating diaphragm, a square wave at the required frequency may be generated by one of the digital ports of the microprocessor 36. A two-pole low pass filter then filters the square wave output. The filter may be for instance a unity gain Sallen-Keys filter with its cut off frequency equal to the square wave frequency. The square wave is made up of the fundamental frequency and the odd harmonics of the fundamental frequency. The filter removes most of the harmonic frequencies and only the fundamental frequency remains. The resulting sinusoidal voltage then feeds the input of the actuating diaphragm.

The sensing diaphragm output voltage is conditioned by a voltage amplifier providing voltage levels suitable for an Analog Digital Converter (ADC) that may be for instance implemented in the microprocessor 36.

Finally, an algorithm implemented in the microprocessor computes the phase shift between the actuation voltage and the sensing voltage from which the viscosity and further the analyte concentration is determined.

### Measurement based on pressure change detection (e.g. pH, Ca⁺⁺, CO₂):

The purpose of chemical sensors consists in converting chemical information into signals suitable for electronic measuring processes. A typical chemical sensor consists of a material-recognizing element and a transducer. An advantageous implementation of such chemical sensors is to use hydrogel thin films as sensing elements. Hydrogels are cross-linked polymers which swell in solvents to appreciable extent. The amount of solvent uptake depends on the polymer structure, and can be made responsive to environmental factors, such as solvent composition, pH value, temperature, electrical voltage etc. Hydrogels are capable to convert reversibly chemical energy into mechanical energy and therefore they can be used as sensitive material for appropriate sensors. Such an approach *per* se has been described for instance by [Guenther07] and [Guenther08] where the transducer of the chemical sensors comprises a piezoresistive silicon pressure sensor forming a Wheatstone bridge 70 (figure 11). It converts the non-electric measuring value into an electrical signal. Various parameters may advantageously be measured using this method such as pH and Ca⁺⁺ concentration.

Also, a measurement concept has been realized by [Herber05] for the detection of carbon dioxide, where the CO₂ induced pressure generation by an enclosed pH-sensitive hydrogel is measured with a micro pressure sensor.

Referring to figure 11, the electrical signal issued by the chemical sensor may be processed after signal conditioning by the analogue to digital converter (ADC) implemented in the microprocessor and the resulting digital values computed by an algorithm that will determine from the pressure, the pH or the concentration of the analyte of interest.

### References:

[Boss09] C. Boss, E. Meurville, J.-M. Sallese, P. Ryser, "Novel chemico-mechanical approach towards long-term implantable glucose sensing", Eurosensors XXIII, Procedia Chemistry, Volume 1, Issue 1, Pages 313-316, 2009.
[Boss11] C. Boss, E. Meurville, P. Ryser, F. Schmitt, L. Juillerat-Jeanneret, P. Dosil-Rosende, D. De Souza, "Multi analyte detection for biological fluids - Towards Continuous Monitoring of Glucose, Ionized Calcium and pH Using a Viscometric Affinity Biosensor", Biodevices, Rome, Italy, January 26-29th 2011.
[Guenther07] Guenther M., Kuckling D., Corten C., Gerlach G., Sorber J., Suchaneck G., Arndt K.-F., "Chemical sensors based on multiresponsive block copolymer hydrogels", Sensors and Actuators B 126 (2007) 97-106.
[Guenther08] Guenther M., Gerlach G., Corten C., Kuckling D., Sorber J., Arndt K.-F., "Hydrogel-based sensor for a rheochemical characterization of solutions", Sensors and Actuators B 132 (2008) 471-476.
[Herber05] S. Herber, J. Bomer, W. Olthuis, P. Bergveld, A. van den Berg, "A Miniaturized Carbon Dioxide Gas Sensor Based on Sensing of pH-Sensitive Hydrogel Swelling with a Pressure Sensor", Biomedical Microdevices 7:3, 197-204, 2005.

### List of references in the drawings:

1 cell culture growth system
   2 incubator
      8 enclosure
      10 shelves
      12 environment control system
   4 culture dish (one or more)
      14 cell culture growth medium
      16 one or more growth medium recipients - (petri dish)
         18 container part
            18a base wall
            18b side wall
         20 cover
   6 cell culture environment monitoring system
      22 sensing device
         28 one or more single parameter or multi-parameter sensors
            28a, 28b sensor probes
               60 analyte sensor
                  61 semi-permeable membrane
                  62 analyte sensitive solution
                  64 analyte to be measured
                  66 microchannel
                  68a, 68b actuating and sensing diaphragms
                  69 anti-biofouling coating
                  63 semiconductor substrate
                  70 pressure sensor wheatstone bridge
         30 signal processing circuitry
            34 RFID transponder
               transponder signal processing circuit
               39 coil (resonance coil L_{R})
            36 MCU
            29 calibration EEPROM
            37 energy storage means
         32 support
            recipient fixing means :
               38 adhesive base; 38' clip
      24 (communications and support) tray
         40 support
            40a bottom surface, 40 top surface, 40c side surface
            46 dish positioning means (recess / protuberances)
         42 antenna
            48 conductor coil/loop/other embedded/mounted/deposited
         44 RFID base station
            signal processing circuit
               50 RFID interrogator
               52 microprocessor
               54 power source
                  memory data logger
               58 communications interface→data transceiver
         47 optical inspection ports
      26 computing and user interface system

## Claims

1. Cell culture environment monitoring system (6) for monitoring parameters relevant to cell growth in at least one culture dish (4) containing a cell growth medium (14), including at least one sensing device (22, 22') configured to measure environmental parameters relevant to cell growth, and a tray (24) supporting said at least one culture dish, **characterized in that** said sensing device comprises an RFID transponder (34) and is configured for mounting inside said culture dish and for immersion at least partially within said cell growth medium for measuring at least one parameter within the cell growth medium, and said tray (24) comprises an RFID base station (44) configured to interrogate the RFID transponder to obtain measurements of said parameters relevant to cell growth, including said at least one parameter within the cell growth medium.

2. Cell culture environment monitoring system according to the preceding claim wherein the tray comprises a support base (40) configured to support and position thereon a plurality of said culture dishes, and the system comprises a plurality of said sensing devices for mounting in the plurality of culture dishes.

3. Cell culture environment monitoring system according to the preceding claim wherein the RFID base station includes an antenna (42) mounted in or on the support base configured to enable communication between the RFID base station and the plurality of sensing devices positioned in the plurality of culture dishes.

4. Cell culture environment monitoring system according to the preceding claim wherein the antenna comprises a conductor loop surrounding the plurality of culture dishes configured for near-field communication with the sensing devices.

5. Cell culture environment monitoring system according to any one of the preceding claims 2-4 wherein the support base comprises optical inspection ports (47) positioned below the culture dishes to allow passage of light through the culture dish and growth medium for optical inspection or testing.

6. Cell culture environment monitoring system according to any one of the preceding claims wherein the RFID base station comprises a signal processing circuit that includes an RFID interrogator (50), a microprocessor (52), a power source (54), a communications interface (58) configured for wireless and/or hardwire link to an external computing and user interface system (26), and optionally a memory for storing or logging data received from the sensing devices.

7. Cell culture environment monitoring system according to any one of the preceding claims wherein the sensing device comprises a plurality of sensors (28) responsive to different ones of said parameters relevant to cell growth.

8. Cell culture environment monitoring system according to any one of the preceding claims wherein the sensing device further includes a microprocessor (36), and energy collecting and/or storage means (37) for short term power supply of the RFID transponder, microprocessor and sensors.

9. Cell culture environment monitoring system according to any one of the preceding claims wherein the sensing device comprises a support or base (32) on which, or within which, sensors (28), and signal processing circuitry (30) are mounted, the support comprising an adhesive base (39) configured to allow the sensing device to be stuck on an inside surface of the culture dish and immersed partially or totally in the cell culture growth medium.

10. Cell culture environment monitoring system according to any one of the preceding claims wherein said parameters relevant to cell growth include temperature.

11. Cell culture environment monitoring system according to the preceding claim wherein said parameters relevant to cell growth further include pH.

12. Cell culture environment monitoring system according to claim 10 or 11 wherein said parameters relevant to cell growth further include any one or more of the parameters selected from the group consisting of Ca⁺⁺, CO₂, glucose, O₂ and light.

13. Cell culture environment monitoring system according to any one of the preceding claims wherein the sensing device comprises sensor probes (28a, 28b) that extend at different lengths, certain said sensor probes (28a) configured for insertion in the cell growth medium and other said sensor probes (28b) configured to remain outside of the growth medium such that parameters within the growth medium and the gaseous environment surrounding the growth medium can be measured.

14. Cell culture growth system comprising a plurality of culture dishes (4) containing a cell growth medium (14) and a cell culture environment monitoring system (6) for monitoring parameters relevant to cell growth in the plurality of culture dishes, including a plurality of sensing devices (22, 22') configured to measure environmental parameters relevant to cell growth in said plurality of culture dishes, and a tray (24) supporting said plurality of culture dishes, wherein each sensing device comprises an RFID transponder (34) and is configured for mounting inside a corresponding said culture dish, and said tray (24) comprises an RFID base station (44) configured to interrogate the RFID transponder to obtain measurements of said parameters relevant to cell growth from said plurality of sensing devices positioned inside the culture dishes.

15. Cell culture growth system according to the preceding claim wherein at least one of said plurality of sensing devices (22, 22') is immersed or partially immersed in the cell growth medium in at least one of said plurality of dishes, for measuring at least one parameter relevant to cell growth within the corresponding cell growth medium.

16. Cell culture growth system according to the preceding claim wherein at least one of said plurality of sensing devices (22, 22') is outside of the cell growth medium in said at least one of said plurality of dishes, for measuring at least one parameter relevant to cell growth outside of the corresponding cell growth medium.

17. Cell culture growth system according to the preceding claim wherein the immersed sensing device is mounted on a bottom wall (18a) of a container part (18) of the culture dish, and the other sensing device is mounted on a cover (20) of the culture dish.

18. Cell culture growth system according to any one of claims 14-17 comprising a cell culture environment monitoring system according to any one of claims 1-13.
